# EUROPÄISCHE PATENTSCHRIFT

(11) **EP 1 709 013 B1**
(45) Veröffentlichungstag und Bekanntmachung des Hinweises auf die Patenterteilung: **10.08.2011**
(21) Anmeldenummer: 05700875.7
(22) Anmeldetag: 13.01.2005
(51) Int. Cl.: C07D 251/64

(54) **KONTINUIERLICHES VERFAHREN ZUR HERSTELLUNG VON HOCHMETHYLOLIERTEM MELAMIN UND VERETHERTEN MELAMINFORMALDEHYDHARZEN**
CONTINUOUS METHOD FOR PRODUCING HIGHLY METHYLOLATED MELAMINE AND ETHERIFIED MELAMINE FORMALDEHYDE RESINS
PROCEDE POUR PRODUIRE EN CONTINU DE LA MELAMINE HAUTEMENT METHYLOLISEE ET DES RESINES DE MELAMINE-FORMALDEHYDE ETHERIFIEES

(30) Priorität: 19.01.2004 DE 102004002849
(43) Veröffentlichungstag der Anmeldung: 11.10.2006
(73) Patentinhaber: BASF SE, 67056 Ludwigshafen (DE)
(72) Erfinder: HITTINGER, Christel, 68199 Mannheim (DE); SCHNEIDER, Jörg, 69469 Weinheim (DE); SCHERR, Günter, 67065 Ludwigshafen (DE); REIF, Martin, 67354 Römerberg (DE); ROBERT, Alain, 67150 Niederkirchen (DE); SCHUPP, Hans, 67549 Worms (DE); EICHFELDER, Andreas, 67133 Maxdorf (DE); HIRSCH, Stefan, 67435 Neustadt an der Weinstrass (DE)
(86) Internationale Anmeldenummer: PCT/EP2005/000259
(87) Internationale Veröffentlichungsnummer: WO 2005/068441

(56) Entgegenhaltungen:
- EP-A- 0 070 924
- EP-A- 0 142 628
- DE-A1- 2 335 299
- GB-A- 1 030 268
- US-A- 4 293 692
- SCHWENK W ET AL: "KOPPLUNGSEFFEKT KNETREAKTOREN FEUR KOMBINIERTE PROZESSE MIT FESTEN UND HOCHVISKOSEN PRODUKTEN" CHEMIETECHNIK, HUTHIG, HEIDELBERG, DE, Bd. 27, Nr. 3, März 1998 (1998-03), Seite 70,72,74, XP000740128 ISSN: 0340-9961
- "Beginn der industriellen Herstellung von Kunststoffverpackungen", Chronik 1960-1979
- Dubbel: "Taschenbuch für den Maschinenbau", 1981 page 955,

## Beschreibung

Die Erfindung betrifft ein Verfahren zur Herstellung von hochmethyloliertem Melamin und von mehrfach mit Alkanol veretherten hochmethylolierten Melaminverbindungen.

Die Herstellung von mehrfach mit Alkanol veretherten mehrfach methylolierten Melaminverbindungen ist allgemein bekannt und beispielsweise in Ullmann's Encyklopädie der technischen Chemie, 4. Auflage, Band 7, S. 403ff beschrieben.

Üblicherweise geht man bei der Herstellung in mehreren Schritten vor: Zunächst wird Melamin mit Formaldehyd in basischem wässrigen Milieu zu mehrfach methylolierten Melaminverbindungen, u.a. Hexamethylolmelamin (HMM) und zu geringen Anteilen entsprechenden Oligomeren, die 2 oder 3 von Melamin abgeleitete Struktureinheiten enthalten, die über Methylen oder Methylenetherbrücken miteinander verbunden sind, umgesetzt. Zu dieser Reaktionsmischung gibt man Alkanol und stellt anschließend den pH-Wert auf 3 bis 5 und verethert die Methylolgruppen des mehrfach methylolierten Melamins bzw. der entsprechenden Oligomere.

Dieses Verfahren ist jedoch mit folgendem Nachteil behaftet: Im ersten Schritt (Methylolierung) wird ein Feststoff (Melamin) in wässriger Formaldehyd-Lösung suspendiert und geht durch die Reaktion in Lösung. Im weiteren Verlauf der Methylolierung entsteht bei der Herstellung höhermethylolierter Melaminformaldehydderivate durch Ausfallen von HMM eine inhomogene teilweise verfestigte Reaktionsmischung. Der Feststoffanteil nimmt in der Regel bei sinkendem Wassergehalt und sinkender Temperatur zu, so dass entsprechende Reaktionsmischungen bei Verwendung hochkonzentrierter Formaldehyd-Lösungen mit klassischen Rühraggregaten im Kessel nicht mehr vollständig durchmischt werden. Insbesondere bei der anschließenden Zugabe des Alkanols fallen weitere Mengen von HMM aus, so dass während der Alkanolzugabe in der Regel das Maximum der Rührerbelastung erreicht wird.

Die Verwendung von Wasser als Verdünnungsmittel oder der Einsatz von niederkonzentriertem Formaldehyd würde sich vorteilhaft auf die Rühr- und Pumpbarkeit der Mischungen auswirken, jedoch würde sich ein derartiges Vorgehen nachteilig auf die sich anschließende Veretherungsreaktion auswirken. Diese lässt sich um so effizienter ausführen, je weniger Wasser in der Reaktionsmischung enthalten ist, da diese "Veretherungsreaktion" eine Gleichgewichtsreaktion mit Wasserbildung auf der Produktseite ist.

Beispielsweise beschreibt die DE-A 23 35 299 ein Verfahren zur Herstellung von veretherten Melaminformaldehydharzen. In Beispiel 3 wird offenbart, dass aus Melamin und Paraformaldehyd kontinuierlich HMM hergestellt wird. Die Veretherung wird in der Weise durchgeführt, dass dieses Produkt in ein Gemisch aus Propanol und Schwefelsäure eingeführt wird. Nach der Veretherung wird das Produkt filtriert und der überschüssige Alkohol verdampft. Nachteilig an diesem Verfahren ist der Einsatz des teueren Paraformaldehyds, das großtechnisch im Vergleich zu wässrigen Formaldehyd-Lösungen nur eine untergeordnete Rolle spielt.

Unter Verwendung von Paraformaldehyd wird ferner im GB Patent Nr. 1 030 268 ein Verfahren zur Herstellung von mit Methanol verethertem HMM beschrieben, wobei der Veretherungsgrad im statistischen Mittel bei 4,85 bis 5,15 liegt. In Beispiel 1 wird ein Herstellungsverfahren offenbart, das neben einer Methylolierungsstufe zwei Veretherungsstufen umfasst.

US 4,293,692 beschreibt ein kontinuierliches Verfahren zur Herstellung von vollständig methylierten, vollständig methylolierten Melaminharzen, wobei die Methylolierungsreaktion in zwei Stufen durchgeführt wird.

Aufgabe der Erfindung war es, ein vereinfachtes Verfahren zur Herstellung von mehrfach methyloliertem Melamin und von mehrfach mit Alkanol veretherten mehrfach methylolierten Melaminverbindungen, insbesondere höhermethylolierten Melaminderivaten wie z.B. Hexamethoxymethylolmelamin, bereitzustellen. Bei dem Verfahren sollte vorteilhaft der Wassergehalt der Reaktionsmischungen so gering wie möglich sein. Ferner sollte das Verfahren vorteilhaft großtechnisch einsetzbar sein und unter Verwendung von kostengünstigen, technisch leicht zugänglichen Einsatzstoffen und einer einfachen, kostengünstigen Verfahrensführung durchführbar sein.

Demgemäss wurde ein Verfahren zur Herstellung von mehrfach methyloliertem Melamin durch Umsetzung von Melamin mit Formaldehyd gefunden, bei dem die Methylolierungsreaktion kontinuierlich in Gegenwart eines Katalysators in einem Knetreaktor durchgeführt wird und Melamin und Formaldehyd in einem Molverhältnis von 1:4 bis 1:10 eingesetzt werden.

Besonders bevorzugt wird Melamin und Formaldehyd in einem Molverhältnis von 1:7 bis 1:10 eingesetzt.

Formaldehyd wird vorteilhaft als eine 35 bis 95 gewichtsprozentige wässrige Formaldehydlösung, insbesondere eine 40 bis 70 gewichtsprozentige wässrige Formaldehydlösung verwendet. Hochkonzentrierte Formaldehydlösungen (40-70 %) können ggf. auch direkt vor dem Eintrag in den Reaktor aus niederkonzentrierten Formaldehydlösungen (20-40 %) generiert werden. Das Melamin wird vorteilhaft als Feststoff eingesetzt. Gegebenenfalls kann das Melamin mit dem Formaldehyd vor dem Einführen in den Knetreaktor gemischt werden.

Die Methylolierungsreaktion kann säuren- oder basenkatalysiert durchgeführt werden. Diese Katalysatoren werden üblicherweise dem gewünschten pH-Wertbereich entsprechend zudosiert. Bevorzugterweise findet eine automatische/automatisierte pHgesteuerte Dosierung der Laugen oder Säuren statt. Bevorzugt werden als Katalysatoren Basen verwendet, vorteilhaft Erdalkali- oder Alkalihydroxide, insbesondere Natrium- oder Kaliumhydroxid in Form ihrer wässrigen Lösungen, oder Alkalimetallsalze wie Natriumcarbonat oder Natriumtetraborat.

Die Methylolierungsreaktion im Knetreaktor wird vorteilhaft bei einem pH-Wert von 6 bis 12, bevorzugt 8 bis 10, durchgeführt. Die Temperatur liegt in der Regel bei 40 bis 120°C. Die Methylolierungsreaktion wird üblicherweise für eine Dauer von 2 bis 90 Minuten durchgeführt, bevorzugt 5 bis 30 Minuten. Erhalten wird vorteilhaft hochmethyloliertes Melamin mit einem Methylolierungsgrad von 4 bis 6, bevorzugt 4,6 bis 5,8. Der Methylolierungsgrad beschreibt das Einbauverhältnis von den Formaldehyd-Gruppen zu dem Melamin-Grundmolekül. Der Methylolierungsgrad nimmt bekanntlich bei gleicher Reaktionszeit mit steigender Reaktionstemperatur bzw. bei gleicher Reaktionstemperatur mit steigender Reaktionszeit zu. Durch Variation dieser beiden Parameter kann der Fachmann durch einfache Vorversuche die Reaktionszeit bzw. -temperatur ermitteln, die zur Erreichung eines bestimmten Methylolierunggrades erforderlich ist. Das methylolierte Melamin ist hochviskos und weist eine teilweise verfestigte Reaktionsmasse auf.

Als Knetreaktoren sind insbesondere einwellige oder zweiwellige heiz- und kühlbare Knetreaktoren, wie beispielsweise Kneter der Firma List oder Buss, geeignet. Bevorzugt sind Kneter mit einer hohen Selbstreinigung der Wellen und Wände, insbesondere solche Kneter mit einer 100 %igen Selbstreinigung. Vorteilhaft findet in den Knetreaktoren keine oder nur eine geringe axiale Rückvermischung statt. Der Leistungseintrag der Kneter liegt vorteilhaft bei 0,1 bis 0,8 kWh/kg.

Die Kneter sind ggf. in mehrere Bereiche aufgeteilt, z.B. in einen Mischungs- bzw. Eintragsbereich, der Dosiereinheiten für Flüssigkeiten und Feststoffe aufweist, in einen Reaktionsbereich, der Verweilzeiten von typischerweise mindestens 2 Minuten bis zu zwei Stunden, bevorzugt bis zu einer Stunde aufweist und in einen Austragsbereich, der Austragsvorrichtungen aufweist.

In dem Reaktionsbereich der Knetreaktoren befinden sich vorteilhaft mehrere Wärmetauscher sowie beispielsweise Rückflussdestillationsapparaturen, die die Temperaturen messen und steuern. Ferner kann über den Brüdenstrom der Wassergehalt in dem Melamin-Formaldehyd-System gesteuert werden.

Der Austrag aus dem Knetreaktor kann beispielsweise über ein in der Höhe verstellbares Überlaufwehr, über ein Auslaufventil, eine Kolbenschleuse oder über eine totraumfreie Einfach- oder Doppelschnecke erfolgen. Bevorzugt ist dabei eine dichtkämmende Anordnung der Schnecke. Der Austrag erfolgt bevorzugt kontinuierlich.

Der Methylolierungsreaktion kann eine Veretherungsreaktion zu mehrfach mit Alkanol verethertem mehrfach methylolierten Melamin folgen. Bei einer nachfolgenden Veretherung wird das mehrfach methylolierte Melamin vorteilhaft kontinuierlich aus dem Knetreaktor ausgetragen.

Vor oder beim Eintragen des mehrfach methylolierten Melamins in das Veretherungsmedium kann das Methylolmelamin gegebenenfalls einem Homogenisierungsschritt unterzogen werden. Die Homogenisierung und Zerkleinerung kann mit Hilfe eines Apparates erfolgen, der nach dem Rotor-Stator Prinzip mit hoher Frequenz arbeitet. Das ausgetragene methylolierte Melamin wird bevorzugt über mehrere aufeinanderfolgende mit hoher Frequenz drehende Rotoren geschickt, wobei mit der Spaltbreite zwischen den Rotoren und den Statoren der Zerkleinerungsgrad einstellbar ist. Geeignete Apparaturen bieten unter anderem die Firmen Ika und Ystral an, beispielsweise der Dispax® (registierte Marke) der Firma Ika.

Nach der Methylolierungsreaktion und gegebenenfalls dem Homogenisierungsschritt gelangt das methylolierte Melamin vorteilhaft kontinuierlich in einen Reaktor, in dem das Alkanol bevorzugt im Überschluss vorliegt. Vorteilhaft sollte das Alkanol in einem Verhältnis zu mehrfach methyloliertem Melamin von mindestens 5-50:1 vorliegen, bevorzugt 10-30:1. Der Veretherungsreaktor kann aus einem oder mehreren Rohrreaktoren, einer Rührkesselkaskade, einem Rührkessel oder aus einer Kombination aus einem oder mehreren Rührkesseln und einem oder mehreren Rohrreaktoren bestehen. Die Veretherung kann auch in getakteten Batchreaktoren, d.h. in parallelen zueinander angeordneten Reaktoren, durchgeführt werden.

Das Alkanol ist vorteilhaft ein C₁- bis C₆-Alkohol oder eine Mischung aus verschiedenen C₁-C₄-Alkanolen, bevorzugt wird als Alkanol Methanol eingesetzt. Die C-Kette der C₁-C₆-Alkanole kann ggf. auch O, N, S, Si-haltige funktionelle Gruppen enthalten. Beispielsweise können funktionelle Gruppen wie Alkanol-, Amino-, Carbonate-, Urethan- und/oder Ester-Gruppen die C-Kette unterbrechen oder endständig eingebaut sein. Der Veretherungsschritt wird bevorzugt säurekatalysiert. Als Säuren kommen Mineralsäuren wie beispielsweise Schwefelsäure oder Salpetersäure, aber auch immobilisierte Säure wie beispielsweise saure Ionenaustauscherharze in Betracht. Der pH-Wert liegt typischerweise bei 1 bis 6, bevorzugt 2 - 5. Die Reaktionstemperatur liegt in der Regel bei 50°C bis zur Siedetemperatur des eingesetzten Alkanols.

Die Veretherung kann gegebenenfalls mehrstufig erfolgen. Der Veretherungsprozess kann zu vollständig oder partiell veretherten mehrfach methylolierten Melaminen führen. Eine Lagerung des partiell veretherten Produktes ist möglich. Zwischen den Veretherungsschritten oder während oder nach einer Lagerung kann Wasser destillativ oder durch Trocknungsmittel (Molsieb, Dinatriumsulfat) oder durch Einsatz von Membranen entfernt werden. An den ersten Veretherungsschritt im Rohrreaktor oder Rührkessel können sich ggf. mehrere weitere Veretherungsschritte auch in unterschiedlichen Reaktoren (Rührkessel oder Rohrreaktor) anschließen. Nach dem letzten gegebenenfalls auch nach vorherigen Veretherungsschritten können sich kontinuierliche Destillationen unter Verwendung von geeigneten Verdampfern (z.B.: Fallfilmverdampfer, Dünnschichtverdampfer, Kessel mit externen Wärmetauschern u.ä.) anschließen. Unter Verwendung eines Rührkessels werden vorteilhaft in einem ersten Schritt nur partiell veretherte Melaminverbindungen erhalten. Nach Bedarf können sich weitere Veretherungsschritte anschließen, die vorteilhaft in einem Rohrreaktor durchgeführt werden.

Das veretherte methylolierte Melamin kann nach Bedarf filtriert und/oder eingeengt und/oder die Viskosität des Produktes durch Zugabe von Lösungsmitteln (z.B. Wasser, C₁-C₆-Alkanole oder aliphatische oder aromatische Kohlenwasserstoffverbindungen) eingestellt werden.

Das auf diese Weise erzeugte veretherte und methylolierte Melamin weist üblicherweise eine Viskosität von 1500 bis 80000, bevorzugt 3000-10000 mPas auf. Das Verhältnis Melamin : veretherten Formaldehydgruppen liegt im Mittel vorteilhaft zwischen 1:4 und 1:6, bevorzugt zwischen 1:4,7 und 1:5,8. Der Monomerengehalt beträgt in der Regel 20 bis 98%, bevorzugt 30 bis 70%. Die Hauptkomponenten sind typischerweise ein- und zweikernige hochmethylolierte und veretherte Derivate, insbesondere Hexamethoxymethylolmelamin.

Die nach diesem Verfahren hergestellten veretherten und methylolierten Melaminverbindungen eignen sich insbesondere als Vernetzungssysteme in Beschichtungsmitteln wie Lacken.

Der Vorteil des erfindungsgemäßen Verfahrens liegt zum einen darin, dass kostengünstige Rohstoffe, d.h. hochkonzentrierte wässrige Formaldehydlösung, verwendet werden können, die bei einer Methylolierungsreaktion in einem Rührkessel zu nicht handhabbaren teilweise verfestigten methylolierten Melaminverbindungen führen würden, zum anderen kann durch die Verwendung von hochkonzentriertem Formaldehyd das bei der Veretherungsreaktion störende Wasser minimiert werden.

Bei dem erfindungsgemäßen Verfahren zur Herstellung von veretherten methylolierten Melaminen kann auf eine Isolierung des methylolierten Melamins verzichtet werden. Ferner kann das Reaktionsvolumen im Vergleich zum Batchprozess reduziert werden. Darüber hinaus lässt sich eine einheitliche Produktqualität leicht steuern und es lassen sich hohe Veretherungsgrade einstellen.

Beispiel:
Ein 250 ml Knetreaktor mit temperierbaren Knetwerkzeugen und Mantel wurde mit einem Zulauf von 394,9 g/h einer 60 gewichtsprozentigen wässrigen Formaldehydlösung (mit 0,73 g Borax versetzt und mit 30 prozentigen Natronlauge auf einem pH-Wert von 8,5-8,8 eingestellt) beschickt. Über die angeschlossene Feststoffdosierung wurden im Mischungsbereich des Kneters 99,6 g/h festes Melamin zudosiert. An den Mischungsbereich schloss sich eine Temperierstrecke an, in der die Mischung auf ca.
60°C vorgeheizt wurde. In der anschließenden Reaktionsstufe, die mit einem Rückflusskühler versehen war, wurde durch Kühlen und Einstellen eines geeigneten Rücklaufverhältnisses die Temperatur auf ca. 100°C gehalten. Nach einer mittleren Verweilzeit von ca. 15 min wurde die Reaktionsmischung mittels einer Austragsschnecke aus dem Reaktor direkt in ein kontinuierlich betriebenes Kesselsystem eingetragen.
Dieser Kessel wurde mit einem Zulauf von 252,8 I Methanol (angesäuert mit 3,5 ml 30 gewichtsprozentiger Salpetersäure) bei 60°C beschickt. Nach einer mittleren Verweilzeit von ca. 30 min (minimale Verweilzeit 15 min) wurde die Mischung in einen nachgeschalteten Rohrreaktor eingetragen, der mit einem Zulauf von 800 ml/h Methanol (pH Wert 3,8, Temperatur 60°C) beschickt wurde. Nach einer mittleren Verweilzeit von 60 min wurde die Mischung direkt auf zwei in Reihe geschaltete Fallfilmverdampfer (p₁ 1bar, p₂ 100 mbar) geführt. Nach Filtration wurde das Produkt per HPLC analysiert: Es enthielt 20 Flächenprozente (HPLC) Hexamethoxymehtylolmelamin, sowie weitere hochmethylolierte/-veretherte Komponenten. Die Gesamtkonzentration der Monomeren lag bei 60 Flächenprozenten (HPLC-GPC).

## Patentansprüche

1. Verfahren zur Herstellung von mehrfach methyloliertem Melamin durch Umsetzung von Melamin und Formaldehyd, **dadurch gekennzeichnet, dass** die Methylolierungsreaktion kontinuierlich in Gegenwart eines Katalysators in einem Knetreaktor durchgeführt wird und dass Melamin und Formaldehyd in einem Molverhältnis von 1:4 bis 1:10 eingesetzt werden.

2. Verfahren nach Anspruch 1, **dadurch gekennzeichnet, dass** der Formaldehyd als eine 35 bis 95 gewichtsprozentige wässrige Formaldehydlösung eingesetzt wird.

3. Verfahren nach den Ansprüchen 1 oder 2, **dadurch gekennzeichnet, dass** die Methylolierungsreaktion bei einer Temperatur von 40 bis 120°C durchgeführt wird.

4. Verfahren nach den Ansprüchen 1 bis 3, **dadurch gekennzeichnet, dass** die Methylolierungsreaktion für 2 bis 90 Minuten durchgeführt wird.

5. Verfahren nach den Ansprüchen 1 bis 4, **dadurch gekennzeichnet, dass** als Knetreaktor ein einwelliger oder zweiwelliger heiz- und kühlbarer Kneter eingesetzt wird.

6. Verfahren nach den Ansprüchen 1 bis 5, **dadurch gekennzeichnet, dass** das methylolierte Melamin ein Methylolierungsgrad von 4,0 bis 6,0 aufweist.

7. Verfahren zur Herstellung von mehrfach mit Alkanolen verethertem mehrfach methylolierten Melamin, **dadurch gekennzeichnet, dass**
(i) die Methylolierungsreaktion gemäß den Ansprüchen 1 bis 6 durchgeführt wird,
(ii) methyloliertes Melamin aus dem Knetreaktor kontinuierlich ausgetragen wird und
(iii) das ausgetragene methylolierte Melamin kontinuierlich in einen Reaktor eingebracht und mit einem Alkanol, das im Überschuss vorliegt, umgesetzt wird.

8. Verfahren nach Anspruch 7, **dadurch gekennzeichnet, dass** der kontinuierliche Austrag gemäß Schritt (ii) mittels eines Überlaufwehrs, eines Auslaufventils, einer Kolbenschleuse oder einer Einfach- oder Doppelschnecke erfolgt.

9. Verfahren nach den Ansprüchen 7 oder 8, **dadurch gekennzeichnet, dass** die Veretherung gemäß Schritt (iii) kontinuierlich durchgeführt wird.

10. Verfahren nach den Ansprüchen 7 bis 9, **dadurch gekennzeichnet, dass** die Veretherung gemäß Schritt (iii) in einer Apparatur ausgewählt aus der Gruppe bestehend aus getakteten Batchreaktoren, einer Kesselkaskade, einem oder mehreren Rohrreaktoren und einem Kessel mit nachgeschaltetem Reaktor durchgeführt wird und zu teil- oder vollverethertem mehrfach methylolierten Melamin führt.

11. Verfahren nach Anspruch 10, **dadurch gekennzeichnet, dass** die Veretherung gemäß Schritt (iii) über mehrere Stufen mit zwischenzeitlicher Destillation zu teil- oder vollverethertem mehrfach methylolierten Melamin führt.

12. Verfahren nach den Ansprüchen 7 bis 11, **dadurch gekennzeichnet, dass** das aus dem Knetreaktor ausgetragene methylolierte Melamin vor dem Eintragen in den Veretherungsreaktor einem Homogenisierungsprozess unterzogen wird.

## Claims

1. A process for preparing polymethylolated melamine by reacting melamine and formaldehyde, which comprises carrying out the methylolation reaction continuously in the presence of a catalyst in a kneading reactor and using melamine and formaldehyde in a molar ratio of from 1:4 to 1:10.

2. The process according to claim 1, wherein the formaldehyde is used in the form of a from 35 to 95% by weight aqueous formaldehyde solution.

3. The process according to claim 1 or 2, wherein the methylolation reaction is carried out at a temperature of from 40 to 120°C.

4. The process according to claims 1 to 3, wherein the methylolation reaction is carried out for from 2 to 90 minutes.

5. The process according to claims 1 to 4, wherein the kneading reactor used is a single-shaft or double-shaft heatable and coolable kneader.

6. The process according to claims 1 to 5, wherein the methylolated melamine has a degree of methylolation of from 4.0 to 6.0.

7. A process for preparing polymethylolated melamine polyetherified with alkanols, which comprises
(i) carrying out the methylolation reaction according to claims 1 to 6,
(ii) continually discharging methylolated melamine from the kneading reactor and
(iii) continuously introducing the discharged methylolated melamine into a reactor and reacting with an alkanol which is present in excess.

8. The process according to claim 7, wherein the continuous discharge of step (ii) is effected by means of an overflow weir, a discharge valve, a lock feeder or a single or twin screw.

9. The process according to claims 7 or 8, wherein the etherification of step (iii) is carried out continuously.

10. The process according to claims 7 to 9, wherein the etherification of step (iii) is carried out in an apparatus selected from the group consisting of switched batch reactors, a tank battery, one or more tubular reactors and a tank having downstream reactor, and leads to partly or fully etherified, polymethylolated melamine.

11. The process according to claim 10, wherein the etherification of step (iii) leads via several stages with intermediate distillation to partly or fully etherified, polymethylolated melamine.

12. The process according to claims 7 to 11, wherein the methylolated melamine discharged from the kneading reactor is subjected to a homogenization process before introduction into the etherification reactor.

## Revendications

1. Procédé de fabrication de mélamine polyméthylolée par mise en réaction de mélamine et de formaldéhyde, **caractérisé en ce que** la réaction de méthylolation est réalisée en continu en présence d'un catalyseur dans un réacteur à malaxage et **en ce que** la mélamine et le formaldéhyde sont utilisés en un rapport molaire de 1:4 à 1:10.

2. Procédé selon la revendication 1, **caractérisé en ce que** le formaldéhyde est utilisé sous la forme d'une solution aqueuse de formaldéhyde de 35 à 95 pour cent en poids.

3. Procédé selon les revendications 1 ou 2, **caractérisé en ce que** la réaction de méthylolation est réalisée à une température de 40 à 120 °C.

4. Procédé selon les revendications 1 à 3, **caractérisé en ce que** la réaction de méthylolation est réalisée pendant 2 à 90 minutes.

5. Procédé selon les revendications 1 à 4, **caractérisé en ce qu'**un malaxeur chauffant et refroidissant à un arbre ou à deux arbres est utilisé en tant que réacteur à malaxage.

6. Procédé selon les revendications 1 à 5, **caractérisé en ce que** la mélamine méthylolée présente un degré de méthylolation de 4,0 à 6,0.

7. Procédé de fabrication de mélamine polyméthylolée polyéthérifiée avec des alcanols, **caractérisé en ce que**
(i) la réaction de méthylolation est réalisée selon les revendications 1 à 6,
(ii) la mélamine méthylolée est déchargée en continu du réacteur à malaxage et
(iii) la mélamine méthylolée déchargée est introduite en continu dans un réacteur et mise en réaction avec un alcanol qui est présent en excès.

8. Procédé selon la revendication 7, **caractérisé en ce que** le déchargement continu selon l'étape (ii) a lieu au moyen d'un déversoir de sortie, d'une soupape de déchargement, d'une vanne à piston ou d'une vis simple ou double.

9. Procédé selon les revendications 7 ou 8, **caractérisé en ce que** l'éthérification selon l'étape (iii) est réalisée en continu.

10. Procédé selon les revendications 7 à 9, **caractérisé en ce que** l'éthérification selon l'étape (iii) est réalisée dans un appareil choisi dans le groupe constitué par des réacteurs discontinus cadencés, une cascade de cuves, un ou plusieurs réacteurs tubulaires et une cuve avec un réacteur connecté en aval, et conduit à une mélamine polyméthylolée partiellement ou totalement éthérifiée.

11. Procédé selon la revendication 10, **caractérisé en ce que** l'éthérification selon l'étape (iii) conduit en plusieurs étapes avec distillation intermédiaire à une mélamine polyméthylolée partiellement ou totalement éthérifiée.

12. Procédé selon les revendications 7 à 11, **caractérisé en ce que** la mélamine méthylolée déchargée du réacteur à malaxage est soumise à un procédé d'homogénéisation avant l'introduction dans le réacteur d'éthérification.
